# EUROPÄISCHE PATENTSCHRIFT

(11) **EP 1 360 306 B1**
(45) Veröffentlichungstag und Bekanntmachung des Hinweises auf die Patenterteilung: **22.11.2006**
(21) Anmeldenummer: 02719712.8
(22) Anmeldetag: 22.01.2002
(51) Int. Cl.: C12N 15/55, C12N 9/20, C12N 15/63, C12R 1/90

(54) **EINE DNA-SEQUENZ DES ENZYMS PHOSPHOLIPASE A1 AUS DEM CILIATEN TETRAHYMENA UND DIE VERWENDUNG DIESER**
DNA SEQUENCE OF THE ENZYME PHOSPHOLIPASE A1 OF CILIATE TETRAHYMENA, AND THE USE OF THE SAME
SEQUENCE D'ADN DE L'ENZYME PHOSPHOLIPASE A1 DU CILIE TETRAHYMENA ET SON UTILISATION

(30) Priorität: 22.01.2001 DE 10105152
(43) Veröffentlichungstag der Anmeldung: 12.11.2003
(73) Patentinhaber: Cilian AG, 48149 Münster (DE)
(72) Erfinder: HARTMANN, Marcus, 48149 Münster (DE); GRENNINGLOH, Marco, 48329 Münster (DE); TIEDTKE, Arno, 48159 Münster (DE)
(74) Vertreter: Meyers, Hans-Wilhelm
(86) Internationale Anmeldenummer: PCT/EP2002/000578
(87) Internationale Veröffentlichungsnummer: WO 2002/057459

(56) Entgegenhaltungen:
- GUBERMAN A ET AL: "A method for the preparation of Tetrahymena thermophila phospholipase A1 suitable for large-scale production." JOURNAL OF APPLIED MICROBIOLOGY, Bd. 86, Nr. 2, Februar 1999 (1999-02), Seiten 226-230, XP002208203 ISSN: 1364-5072
- HARTMANN M ET AL: "Screening for and characterization of phospholipase A1 hypersecretory mutants of Tetrahymena thermophila." APPLIED MICROBIOLOGY AND BIOTECHNOLOGY, Bd. 54, Nr. 3, September 2000 (2000-09), Seiten 390-396, XP002208204 ISSN: 0175-7598
- DATABASE WPI Derwent Publications Ltd., London, GB; AN 1998-391046 XP002208205 R. KOISHI ET AL: "Aspergillus-derived phospholipase A1 gene - used for the recombinant production of phospholipase A of high purity and in a high yield" & JP 10 155493 A (SANKYO CO LTD), 16. Juni 1998 (1998-06-16)

## Beschreibung

Die Erfindung betrifft eine Nukleinsäure kodierend für die Phospholipase A1 und die Verwendung dieser entsprechend dem Oberbegriff des Anspruchs 1 bis 5.

Hefen, Bakterien und Mammalia-Zellen haben für die biotechnologische Darstellung und Produktion rekombinanter Wirkstoffe durch die heterologe Expression von Fremdproteinen eine große Bedeutung. Hierbei werden, bakterielle Expressionssysteme auf der Basis von *E. coli* oder *B. subtilis* zur Produktion von rekombinanten Peptiden bzw. Proteinen wie z.B. Insulin, Interleukin-2, Gewebe-Plasminogen-Aktivator, Proteasen und Lipasen verwendet. Bei gram-negativen Bakterien basieren die Expressionssysteme meist auf der Verwendung von genetischen Elementen, wie dem lac-Operon oder dem Tryptophan-Operon. Dabei werden die wirtsfremden Proteine entweder in "inclusion bodies" (Einschlußkörper) innerhalb der Zelle produziert, oder, bei Verwendung von Expressionsystemen auf der Basis von β-Lactamase-Genen, in den periplasmatischen Raum. Die Produktion von rekombinanten Proteinen in das umgebende Fermentationsmedium ist nicht etabliert. Bei gram-positiven Bakterien werden bisher fast ausschließlich nur zelleigene Proteine in Expressionssysteme eingebaut und exprimiert.
Hefen, wie *S. cerevisiae, Hansenula polymorpha, Kluyveromyces lactis* oder *Pichia pastoris* werden ebenfalls zur heterologen Expression rekombinanter Proteine, wie z.B. von humanem Faktor XIIIa, bovinem Pro-Chymosin, Phytase oder Oberflächenantigenen eingesetzt. Die Expressionssysteme basieren hier auf Shuttle-Vektoren (Vektor mit einem Hefe- und einem bakteriellen Anteil), die (je nach Hefe-Art) auf den genetischen Elementen der Galakto-Kinase-Epimerase, der Methanol-Oxidase, der sauren Phosphatase oder Alkoholdehydrogenase aufgebaut sind. In der Regel wird das rekombinante Protein in das Cytoplasma der Zelle produziert. Bei Verwendung von hefeeigenen Signalsequenzen, wie der des Alpha-Faktors, können die exprimierten Proteine auch in das Fermentationsmedium sezerniert werden. Die Glykosylierung sezernierte Proteine erfolgt nach dem "high-mannose"-Typ, häufig treten Hpyerglycosilierungen am Protein auf, die beim Patienten zur Bildung von Antikörpern führen kann.
Mammalia-Zellen, wie verschiedene Zelltypen von Nagern (CHO-Zellen, C127-Zellen) oder Affen (Vero-, CV-1- oder COS-Zellen) kommen ebenfalls zur heterologen Expression rekombinanter Proteine zum Einsatz. Die Expressionsysteme basieren hier auf rekombinanten Viren (BPV-Vektor) oder auf Shuttle-Vektoren. Zur Regulation der Expression werden virale SV40-Enhancer/Promotor-Systeme oder zelluläre Verstärker-Elemente eingesetzt. Die rekombinanten Proteine, wie Erythropoietin, werden in das Fermentationsmedium sezerniert, da die Fremdgene in der Regel bereits Signalsequenzen mitbringen und diese vom Expressionssystem verstanden und zur Zielsteuerung verwendet werden.
Zur biotechnologischen Produktion von glykosylierten, extrazellulären Enzymen werden desweiteren Protozoen der Gattung *Tetrahymena* eingesetzt. *Tetrahymena* wächst auf kostengünstigen Fermentationsmedien unter Einsatz von Standartfermentationsverfahren. Zur Transformation solcher *Tetrahymena*-Zellen stehen Vektoren zur Verfügung, die auf den rDNA-Elementen von *Tetrahymena* basieren. Zur heterologen Expression von bakteriellen Proteinen in *Tetrahymena* werden DNA-Konstrukte aus Genen von *Tetrahymena* eingesetzt. Wenn geeignete genetische Elemente zur Regulation der Transkription, der Zielsteuerung und der Glykosilierung von Fremdproteinen zur Verfügung stehen, ist *Tetrahymena* ein ideales Expressionssystem zur kostengünstigen Produktion therapeutischer rekombinanter Proteine.

Die bisher verwendeten Gram-negativen bakteriellen Expressionsysteme führen in der Regel zur Bildung von "inclusion bodies" in der Zelle, einhergehend mit einer Denaturierung der Proteine. Für die Gewinnung des rekombinanten Proteins müssen die Zellen aufgebrochen und das denaturierte, inaktive Protein für seine Funktion zurückgefaltet werden. Dies verursacht zusätzliche kostenintensive Verfahrensschritte und erniedrigt die Ausbeute des erwünschten Proteins. Die für eukaryontische Proteine wichtige Gkykosylierung unterbleibt völlig. Bei der Verwendung von Gram-positiven bakteriellen Expressionsystemen ist der Abbau des Zielproteins durch hohe proteolytische Aktivitäten in der Fermentationsbrühe zudem problematisch.

Bei der Verwendung von Hefen zur heterologen Expression wird das erwünschte Zielprotein ebenfalls nur in die Zelle produziert, aus der es durch Zellaufschluss entfernt werden muss. Dies verursacht, wie bei bakteriellen Expressionssystemen, zusätzliche zeit- und kostenintensive Verfahrensschritte. Werden hefeeigene Signalpeptide verwendet, so werden die Fremdproteine bei der Sezernierung nicht korrekt gespleißt und glykosyliert.

Werden hingegen Mammalia-Zellsysteme zur Produktion von rekombinanten Proteinen eingesetzt, so liegen die erwünschten Proteine extrazellulär, korrekt gespleißt und glykosyliert im Fermentationsmedium vor. Nachteilig ist hier jedoch zum einen die niedrige Expressionrate durch die fehlerhafte Prozessierung und ineffiziente Translation von Genen, die über virale Vektoren in das Genom der Produktionszellinie eingebracht werden müssen. Zum anderen sind die serumhaltigen Fermentionsmedien für Mammalia-Zellen extrem kostenintensiv. Zudem ist die Fermentationstechnologie für die scherkraftempfindlichen Zellinien auf Grund von Konstruktionen zur blasenfreien Belüftung aufwendig und ebenfalls kostspielig. Weiterer Probleme ergeben sich durch die hohe Gefahr der Infektion der Zelllinien durch Mycoplasmen und Viren.

Zusammengenommen hat die Verwendung von Mammalia-Zellen zur biotechnologischen Herstellung von rekombinanten Proteinen sehr hohe Kosten, Sicherheitsauflagen und niedrige Ausbeuten zur Folge.

Für den Einsatz von Ciliaten, wie *Tetrahymena* gelten die oben genannten Nachteile bei der Produktion rekombinanter Proteine nicht. So werden beispielsweise einige saure Hydrolasen, die an der Verdauung von Nahrungspartikeln beteiligt sind, in großen Mengen und komplex glykosiliert aus der Zelle ausgeschleust.

Alam et al. beschreiben im J. Euk. Microbiol. 43 (4), 1996, Seiten 295 bis 303 die Klonierung eines Gens, das für die saure alpha-Glucosidase von *Tetrahymena pyriformis* kodiert. Das Protein wird jedoch nur zu einem geringen Teil aus der Zelle ausgeschleust. Desweiteren ist in der internationalen Patentanmeldung PCT/EP00/01853 das Gen einer β-Hexosaminidase aus *Tetrahymena thermophila* beschrieben, von dem jedoch bekannt ist, dass es nur zu ca. 80 % aus der Zelle ausgeschleust wird.

Bisher ist es jedoch nicht möglich, glykosylierte eukaryontische Proteine in *Tetrahymena* zur Expression zu bringen, die gleichfalls ausschließlich in das Fermentationsmedium sezerniert werden. Ursache ist, das bisher die DNA-Sequenzen von *Tetrahymena-*eigenen, extrazellulären Proteinen unbekannt waren, die für die Konstruktion von Expressionsvektoren notwendig sind und die ausschließlich das wirtsfremde Protein in das umgebende Fermentationsmedium ausschleusen. Bekannt sind die DNA-Sequenzen eines Proteins, dass für die β-Hexosaminidase von *Tetrahymena thermophila* codiert. Eine solche Sequenz ist unter den amtlichen Kennzeichen DE 199 58 979.8, DE 199 09 189.7 sowie unter PCT/EP00/01853 zum Patent angemeldet worden. Nachteil dieser Sequenzen ist jedoch, dass die sie enthaltenen Prä/Propeptide ein wirtfremdes Protein nur zu ca. 80 Prozent in das umgebende Fermentationsmedium steuern. Ursache ist, dass das Enzym β-Hexosaminidase unter natürlichen Umständen zu ca. 20 Prozent membranständig vorliegt und nur etwa 80 Prozent des natürlich produzierten Enzyms aus der Zellegeschleust werden. Aus diesem Grund lenken die Prä/Pro-Peptide der β-Hexosaminidase, wenn sie durch gentechnische Verfahren vor ein wirtsfremdes Protein positioniert werden, dieses wirtsfremde Protein zu ca. 20 Prozent in die Cytoplasmamembran auf der Oberfläche von *Tetrahymena thermophila.* Dies ist von erheblichen verfahrenstechnischem Nachteil für die rekombinante Wirkstoffherstellung. Zum einen veringert sich so die Ausbeute, da ein Teil des exprimierten Proteins membrangebunden in den Zellen verbleibt und so nicht das gesamte exprimierte Protein aus der Fermenterbrühe aufgereinigt werden kann. Zum anderen kann das wirtsfremde Protein in der Zellmembran toxische Effekte auf die Wirtszellen haben und so das Zellwachstum verlangsamen.

Des weiteren sind bis heute keine konstitutiven Promotoren von *Tetrahymena* bekannt, die eine gleichmäßige bzw. ständige Transkription von heterologen Proteinen bewirken. Bisher sind lediglich Promotoren von Histon- und Tubulin-Genen bekannt (Bannon et al., 1984, Gaertig et al., 1993). Ein entscheidender Nachteil dieser Promotoren ist jedoch, dass diese abhängig vom Zellzyklus aktiviert werden. Gene heterologer Proteine, die mit solchen zellzyklusabhängigen Promotoren verbunden sind, werden nur in wachsenden bzw. sich teilenden Zellen zur Expression gebracht. Dies hat erhebliche verfahrenstechnische Nachtteile, da das gewünschte Protein so nur in der logarithmischen Wachstumsphase produziert wird. In der stationären Wachstumsphase, in der im Produktionsprozess die höchste Zelldichte und damit die höchste Leistungsfähigkeit des Expressionsorganismus (*Tetrahymena*) erreicht wird, findet kaum noch Zellwachstum und damit nur eine geringe Expression des heterologen Proteins statt.

Aufgabe der Erfindung ist es, Ein System bereitzustellen, das es ermöglicht, in einem Expressionsystem heterologe Proteine nach der Transformation in *Tetrahymena* aus den Zellen in das Fermentationsmedium zu produzieren.

Die Aufgabe wird durch ein System gelöst, bei dem eine Nukleinsäure mit einer Sequenz mit der Seq. ID. Nr. 1 codierend für eine Phospholipase A1 (Seq. ID Nr. 7) Verwendung findet. Vorteilhafterweise wird das Expressionsprodukt dieser DNA unter Kulturbedingungen in großen Mengen aus der Zelle ausgeschleust. Das exprimierte Protein wird in hohem Maße in das umgebene Kulturmedium ausgeschleust und liegt nicht membranständig vor. Die erfindungsgemäße Nukleinsäuresequenz enthält einen Promotor, der eine konstitutive, d.h. zellzyklus-unabhängige Transkription der ihr nachgeschalteten Gene heterologer Proteine bewirkt. Die konstitutive Transkription hat dabei den Vorteil, dass die Proteine ständig, ohne Einfluss des Zellzyklus, im Wirtorganismus heterolog exprimiert werden. Somit kann auch während der stationären Wachstumsphase mit geringen Zellwachstum die Transkription des Fremdgens erfolgen und das heterologe Protein exprimiert werden.

Die erfindungsgemäße DNA-Sequenz der Phospholipase A₁ beinhaltet vorzugsweise einen Upstreambereich der PLA₁ (Seq. ID Nr. 2), der die Promoterelemente für die Inititiation der Transkription trägt, Signal- und ein Propeptid, weitere genetische Elemente zur Zielsteuerung von Proteinen sowie insbesondere einen Downstreambereich der PLA₁ (Seq. ID Nr. 3), der genetische Elemente für die Termination der Transkription enthält. Die Verwendung dieser Nukleinsäuren in einem Vektor ermöglicht es, heterolog exprimierte Proteine unabhängig vom Zellzyklus zu exprimieren und sie gezielt aus der Zelle in das umgebende Kulturmedium zu transportieren, ohne dass dabei exprimierte Proteine in die Cytoplasmamembran eingebaut werden, wodurch diese somit ohne Zellaufschluss aus der Fermentationsbrühe isolierbar sind.

Fig. 1 zeigt eine Nukleinsäure kodierend für den Upstreambereich (Seq. ID Nr. 2), den codierenden Bereich (Seq. ID Nr. 1) und den Downstreambereich (Seq. ID Nr. 3) der Phospholipase A₁ aus Ciliaten.
Fig. 2 zeigt ein entsprechendes Expressionsprodukt der Nukleinsäure gemäß Seq. ID. Nr. 1. Das Protein gemäß Seq. ID. Nr. 7 ist ebenfalls Gegenstand der vorliegenden Erfindung.

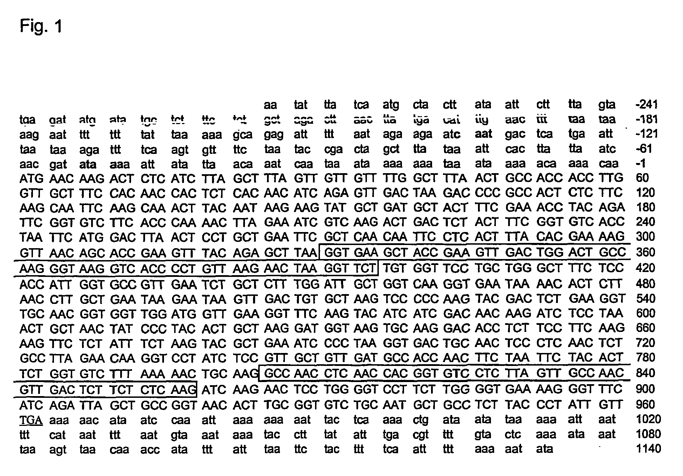

Gegenstand der Erfindung ist insbesondere auch die Signalsequenz (Seq. ID Nr. 6) des erfindungsgemäßen Proteins. Es handelt sich dabei bevorzugt um die Aminosäuren 1 bis 110 des erfindungsgemäßen Proteins (Seq. ID Nr. 5). Auch eine Nukleinsäure, die für das N-terminale Fragment kodiert, ist Gegenstand der Erfindung (Seq. ID Nr. 3). Es handelt sich dabei bevorzugt um ein Fragment der erfindungsgemäßen Nukleinsäuren (Seq. ID Nr. 4), insbesondere mit der Nukleinsäuresequenz 1 bis 155 gemäß Fig. 1.
Die Nukleinsäuresequenz des nichttranslatierenden Bereiches (UpstreamBereich) (Seq. ID Nr. 2) oberhalb des kodierenden Sequenzbereichs der PLA₁ aus *Tetrahymena* liegt zwischen Position -275 und Position -1 (in Kleinbuchstaben dargestellt). Der ermittelte nichttranslatierte Bereich umfasst 275 Basen. Als Elemente eines Promotors liegt eine TATA-Box auf Position -49 bis -55 (fett gedruckt), sowie eine putative CAAT-Box zwischen Base -133 und Base -136 (fett gedruckt). Der kodierende Sequenzbereich der cDNA ist in Grossbuchstaben dargestellt. Mit dem Startcodon ATG beginnt die Numerierung der Sequenz. Aus der Proteinsequenzierung bekannte Bereiche sind umrandet und das Stopcodon unterstrichen. Das reife Protein wird ab Base 331 codiert. Das Sequenzprotokoll von der Base 1 bis zur Base 330 stellt die Prä/Pro-Sequenz (Seq. ID Nr. 8) der PLA₁ dar. Das Sequenzprotokoll von Base 331 bis Base 963 stellt die Sequenz der reifen PLA₁ (Seq. ID Nr. 9) dar. In Position 961 befindet sich der Translationsstop TGA, in Position 1039 das Polyadenylierungssignal AAT AAA. Die Nukleinsäuresequenz von Position 964 bis Position 1134, die unterhalb der kodierenden Sequenz der PLA₁ von *Tetrahymena* liegt, stellt den Downstream-Bereich der PLA₁ (Seq. ID Nr. 3) dar, der nicht translatiert wird (ebenfalls in Kleinbuchstaben dargestellt). In Position 964 bis Position 1101 liegt der aus Sequenzierung der cDNA bekannte Bereich, der ebenfalls mit inverser PCR bestätigt wurde. Dem letzten Codon der mRNA (ttt, Position 1098-1101) wird nach der Transkription der Poly-A-Schwanz angehängt.

Ein weiterer Aspekt der Erfindung ist die Verwendung einer Nukleinsäuresequenz von sauren Hydrolasen gemäß der Erfindung oder Teilen davon zur homologen oder heterologen Expression von rekombinanten Proteinen und Peptiden, sowie zur homologen oder heterologen Rekombination ("knock-out", "gene replacement").

Gegenstand der Erfindung ist ein Verfahren zur homologen oder heterologen Expression von Proteinen und Peptiden, sowie zur homologen oder heterologen Rekombination ("knock-out", "gene replacement"), wobei Ciliaten mit einer erfindungsgemäßen Nukleinsäure transfiziert sind.

Die Nukleinsäuren oder Teile können insbesondere mit den für homologe oder heterologe Expression von Proteinen üblichen Enhancer, Promotoren, Operatoren, Origins, Terminatoren, Antibiotikaresistenzen oder anderen Nukleinsäuren oder DNA-Fragmenten oder Sequenzen jeglicher Art von Viroiden, Viren, Bakterien, Archezoen, Protozoen, Pilzen, Pflanzen, Tieren oder Menschen kombiniert werden.

Die erfindungsgemäße Nukleinsäure ist insbesondere in einem Vektor, einem Plasmid, einem Cosmid, einem Chromosom oder Minichromosom, einem Transposon, ein IS-Element, einer rDNA oder jeder anderen Art ringförmiger oder linearer DNA oder RNA enthalten.

Gegenstand der Erfindung ist auch ein Verfahren, bei dem die für Phospholipase A1 kodierende erfindungsgemäße Nukleinsäure oder Teile davon mit den für homologe oder heterologe Expression üblichen Enhancer, wie z.B. der NF-1 Region (ein Cytomegalovirus Enhancer) Promotoren, wie z.B. die *lac,trc, tic* oder *tac*-Promotoren, die Promotoren der Klasse II und III des T7 RNAP-Systems, Bacteriophage T7- und SP6-Promotoren, *aprE-,* Amylase oder *spac*-Promotoren für *Bacillus-*Expressionssysteme, AOX1- AUG1 und 2 oder GAPp-Promotoren (*Pichia*) für Hefe-Expressionsysteme, RSV-Promotor (SV40-Virus), CMV-Promotor (Cytomegalovirus), AFP-Promotor (Adenoviren) oder Metallothionin-Promotoren für Mammalia-Expressionssysteme, Sindbis Virus-Promotoren oder Semlike-Forest Virus Promotoren für Insektenzellen, Promotoren für Insektenzellexpressionssysteme, wie hsp70, DS47, Actin 5C oder Copia, pflanzenspezifische Promotoren, wie 35S Promotor (Cauliflower mosaic virus), Amylase-Promotor oder Klasse I Patatin Promotor, Operatoren, wie z.B. dem *tet*-Operator, Signalpeptiden, wie a-MF praepro Signalsequenzen (Saccharomyces), Origins, Terminatoren, Antibiotika- und Wirkstoffresistenzen, wie Ampicilin, Kanamycin, Streptomycin, Chloramphenicol, Penicillin, Amphotericin, Cycloheximid, 6-Methylpurin, Paromomycin, Hygromycin, α-Amanatin, Auxotrophie-Marken, wie dem Gen der Dihydrofolat Reduktase oder anderen Nukleinsäuren bzw. DNA-Fragmenten bzw. Sequenzen jeglicher Art von Viroiden, Viren, Bakterien, Archezoen, Protozoen, Pilzen, Pflanzen, Tieren oder Menschen kombiniert werden.

Insbesondere wird die erfindungsgemäße Nukleinsäure oder Teile davon in einen Vektor, ein Plasmid, ein Cosmid, ein Chromosom oder Minichromosom, ein Transposon, ein IS-Element, eine rDNA oder jede andere Art ringförmiger oder linearer DNA oder RNA eingebaut.

Dem Fachmann ist klar, dass erfindungsgemäß auch Nukleinsäuren eingesetzt werden können, die zumindest 40% homolog mit der Nukleinsäure gemäß Seq. ID. Nr. 1 ist. Auch das Protein gemäß Seq. ID. Nr. 2 kann modifiziert werden ohne die Funktion zu verlieren. So können beispielsweise sogenannte konservative Austausche von Aminosäuren durchgeführt werden. Dazu können z.B hydrophobe Aminosäuren untereinander ausgetauscht werden.

Zur Aufreinigung und Isolierung der Phospholipase A₁ aus *Tetrahymena* und zur Bestimmung der Sequenz können folgende Methoden verwendet werden.

### Gewinnung von PLA₁

PLA₁ wurde aus zellfreien Kulturüberständen von *Tetrahymena thermophila* gewonnen. Hierzu wurden die Zellen in einem 2-L-Fermenter (Biostat MD, Braun Diessel Biotech, Melsungen, Deutschland), der über eine digitale DCU-Kontrolleinheit gesteuert wurde, fermentiert. Dabei wurde der Fermenter zunächst für 24 Stunden im Batchbetrieb und anschließend kontinuierlich gefahren. Ein Abernten des zellfreien Kulturüberstandes wurde über ein Perfusionsmodul mit einer Porengröße von ca. 0,3 µm (S6/2, Enka, Wuppertal) gewährleistet.
Die Fermentation wurde unter folgenden Parametern gefahren:
- das Arbeitsvolumen betrug 2 Liter
- die Perfusionsrate betrug 2 Liter/Tag
- die Rührerdrehzahl wurde auf 800 Upm begrenzt
- die Temperatur lag konstant bei 30°C
- der pH-Wert wurde konstant bei pH 7 gehalten
- der Animpftiter lag bei 50.000 Zellen/ml

Für die Fermentation wurde der Stamm SB 1868 VII verwendet. Hierbei handelt es sich um einen Wildtyp von *Tetrahymena thermophila.*

Die Fermentation wurde über einen Zeitraum von 264 Stunden durchgeführt und die Ernten auf PLA₁-Aktivität getestet.

### Aufreinigung der PLA₁

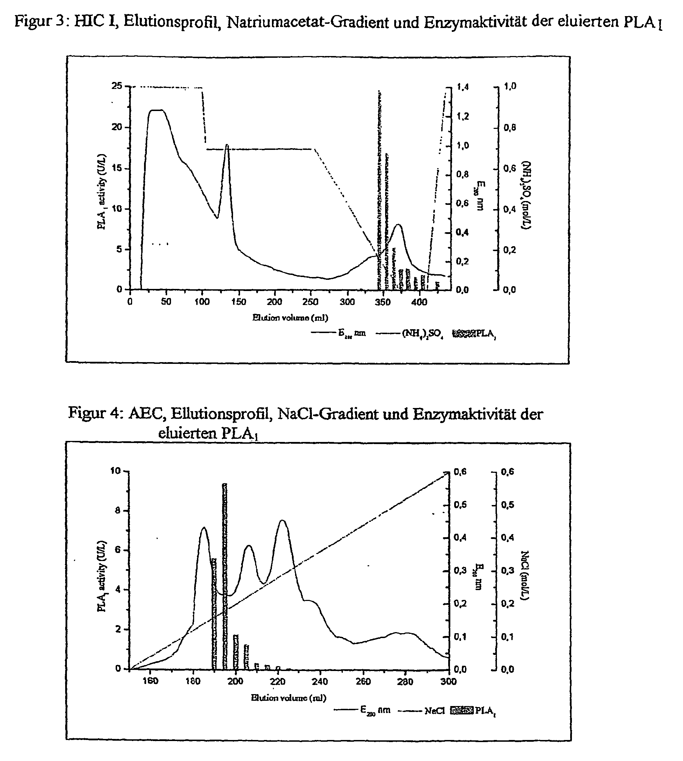

Für die Aufreinigung der PLA₁ wurde 1Liter zellfreier Kulturüberstand aus der Fermentation verwendet. Dieser wurde mit 140 g Ammoniumsulfat versetzt und über eine Ultrafiltrationseinheit (Pellikon XL, Ausschlussgrösse 3kDa, Millipore) auf ein Volumen von 50ml eingeengt. Anschliessend wurde die Probe über eine Hydrophobe Interaktionschromatographie (20 x 1,6 Fractogel EMD Phenyl 1 650, Merck, Darmstadt) aufgereinigt. Dabei betrug die Flussrate 5 ml/min und das Eluat wurde in 5ml Fraktionen aufgefangen. Die Enzymaktivität wurde über die Deacylierung eines radioaktiv markierten Phospholipids (L-3-Phosphatidylcholine, 1-palmitoyl-2-[1-¹⁴C]linoeloyl) gemessen. Fig. 3 zeigt das erhaltene Elutionsprofil, den Natriumacetat-Gradienten und die Enzymaktivitäten in den einzelnen Fraktionen.

Die drei Fraktionen mit den höchsten Enzymaktivitäten wurden zusammengefasst und mit Hilfe einer Ultrafiltrationseinheit in den Startpuffer (Bis-Tris 20mM, pH 6,5) für die Anionenaustauscherchromatographie (AEC) umgepuffert. Anschließend wurde die Probe auf die Säule aufgetragen (Q-Sepharose-Hiload-16/10, Pharmacia, Schweden) und die PLA₁ mit einem linearen NaCl-Gradienten (Flussrate= 3ml/min) von der Säule eluiert und in 5ml Fraktionen aufgefangen. Fig. 4 zeigt das erhaltene Elutionsprofil, den NaCl-Gradienten und die Enzymaktivitäten der einzelnen Fraktionen.

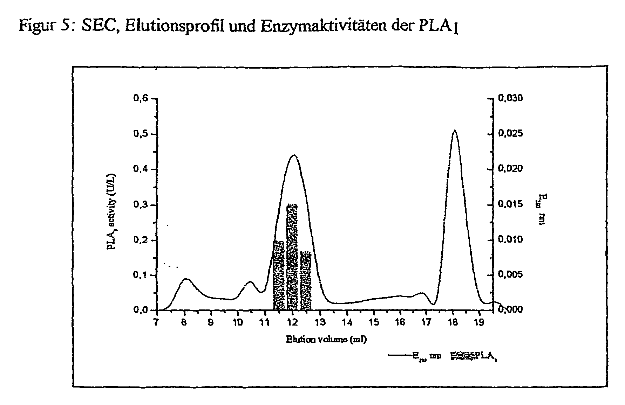

Aus der Fraktion mit der höchsten PLA₁ Aktivität wurden 200µl entnommen und über Grössenausschlusschromatographie (SEC) aufgetrennt. Hierfür wurde eine Superdex HR 75 30/10 Säule (Pharmacia, Schweden) verwendet. Die Flussrate betrug bei dieser Chromatograpie 0,6 ml/min, das Eluat wurde in 200µl Fraktionen aufgefangen. Fig. 5 zeigt das erhaltene Elutionsprofil und die Enzymaktivitäten der einzelnen Fraktionen.

Die erhaltenen Fraktionen wurden auf Ihre Reinheit mit Hilfe der eindimensonalen Gelelektrophorese untersucht. Dabei wurden zwei distinkte Banden bei ~26 und ~28 kDa ermittelt. Eine Auftrennung dieser beiden Banden durch zweidimensionale Gelelektrophorese ergab eine Aufspaltung beider Banden in 2 bzw. 3 Spots mit unterschiedlichem isoelektrischen Punkten. Diese lagen bei den 26 kDa Proteinen bei pH 6,3 und 5,7, bei den 28 kDa Proteinen bei pH 6,3, 5,7 und 5,3. Eine anschliessende Untersuchung dieser Spots über eine Mass-Fingerprint-Analyse ergab, das es sich bei diesen Spots um Isoformen des gleichen Proteins handelt.

### Molekularbiologische Untersuchung der PLA₁

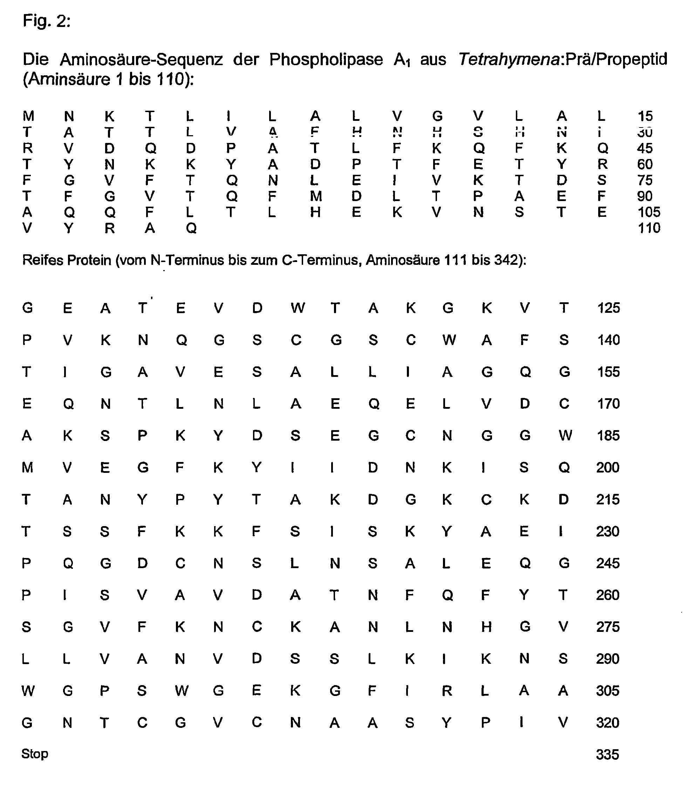

Nachdem die Reinheit des Proteins nachgewiesen war, wurden Proben des Proteins auf PVDF-Membran geblottet und N-Terminal ansequenziert. Zusätzlich wurde eine weitere Probe mit der tryptisch verdaut und ebenfalls ansequenziert. Mit Hilfe der dabei gewonnenen Proteinsequenzen wurden Oligonucleotidprimer hergestellt und diese dann in der Reversen-Transkriptase-PCR (3'RACE, Rapid Amplifikation of cDNA Ends) eingesetzt. Über diese PCR-Technik gelang es die cDNA für die Phospholipase A₁ zu amplifizieren und anschließend zu sequenzieren. Die erhaltene Sequenz hat eine Länge von 633 Basen bzw. 729 Basen, das davon abgeleitete Molekulargewicht des reifen Proteins beträgt ca. 22,4 kDa. In der deduzierten Sequenz fanden sich die aus der Proteinsequenzierung ermittelten Oligopeptide von 22 Aminosäuren (N-Terminal) bzw. 18 Aminosäuren (innerhalb des Proteins) zu 100% wieder. Zusätzlich zur Sequenz des reifen Proteins konnte auch die Sequenz des Prä/Pro-Peptids mit Hilfe der 5' RACE (Rapid Amplifikation of cDNA Ends) editiert werden (Fig. 2). Hierbei handelt es sich um ein 110 Aminosäuren langes Peptid, das zum einen die Signalsequenz und zum anderen das Propeptid trägt, dass das Enzym inakiviert und erst am endgültigen Wirkungsort des Enzyms abgespalten wird. Sequenzvergleiche ergaben bis auf ein Consensussequenz von 5 Aminosäure (GxSxG), die in allen Lipasen und Phospholipasen vorkommt und als Bindungsstelle für Lipide bzw. Phospholipide diskutiert wird, keine Homologien zu bereits bekannten Phospholipasen A₁, Des weiteren wurde über inverse PCR die Upstream- und Downstream-Sequenzen der PLA₁ ermittelt (Fig. 1). Dazu wurde genomische DNA mit Restriktionsendonukleasen geschnitten, mit T4 Ligase ligiert und schliesslich mit inversen Primern ampliziert. Für die Amplizierung des Upstream Bereiches der PLA₁ über inverse PCR wurde mit der Restriktionsendonuklease Ssp I geschnittene genomische DNA verwendet. Dabei konnte upstream ein Bereich von 275 Basen editiert und Promotorelemente identifiziert werden. Im Position -136 liegt eine CAAT-Box, die einen vergleichbaren Abstand vom Translationsstart, wie die von Brunk und Sadler (1990) gefundenen CCAAT-Boxen der Histon-Gene (-141 bzw. -151) von *Tetrahymena* haben. Eine TATA-Box, die bei eukaryontischen Genen den exakten Startpunkt der Transkription festlegt, wurde auf Position -55 identifiziert. Ihre Sequenz entspricht der bei Eukaryonten gefundenen Consensus-Sequenzen. Für die Amplifizierung des Downstream-Bereiches über inverse PCR, der den Terminator für die Transkription der PLA₁ aus *Tetrahymena* enthält, wurde mit Bam H1 geschnittene genomische DNA verwendet. So konnten zusätzlich zu dem aus der 3'RACE bekannten downstream-Bereich weitere 222 Basen ermittelt werden (Fig. 3).

## Patentansprüche

1. Nukleinsäure aus kodierenden und nicht-kodierenden Bereichen mit der Seq ID. Nr. 4, wobei die kodierenden Bereiche die Phospholipase A₁ (PLA₁) von *Tetrahymena thermophila* (Seq ID. Nr. 5) kodieren und die nicht-kodierenden Regionen die upstream- und downstream Bereiche dieses Gens darstellen.

2. Nukleinsäure nach Anspruch 1, wobei die nicht-codierenden upstream- und downstream Bereiche die Seq. ID Nr. 2 und 3 aufweisen und die codierende Regionen die Seq. ID Nr. 1, 8 und/oder 9.

3. Nukleinsäure mit einer der Sequenzen Seq. ID No. 1, 2, 3, 8 oder 9.

4. N-terminale Signalsequenz der PLA₁ (Seq. Id. No. 5) gemäß Anspruch 1, mit der Sequenz Seq ID Nr. 6.

5. Phospholipase A₁ Protein (PLA₁) mit der Aminosäuresequenz gemäß Seq. ID Nr 7.

6. Verwendung einer Nukleinsäure gemäß den Ansprüchen 1 bis 3 zur homologen oder heterologen Expression von rekombinanten Proteinen und Peptiden, sowie zur homologen oder heterologen Rekombination ('knock-out", "gene replacement").

7. Verfahren zur homologen oder heterologen Expression von Proteinen und Peptiden, sowie zur homologen oder heterologen Rekombination ("knock-out", "gene replacement"), wobei Ciliaten mit einer Nukleinsäure gemäß einem der Ansprüche 1 bis 3 transfiziert sind.

8. Verfahren nach Anspruch 7, wobei die Nukleinsäuren oder Teile davon mit den für homologe oder heterologe Expression von Proteinen üblichen Enhancer, Promotoren, Operatoren, Origins, Terminatoren, Antibiotikaresistenzen oder anderen Nukleinsäuren oder DNA-Fragmenten oder Sequenzen jeglicher Art von Viroiden, Viren, Bakterien, Alchezoen, Protozoen, Pilzen, Pflanzen, Tieren oder Menschen kombiniert werden.

9. Verfahren nach Anspruch 7 und/oder 8, wobei die Nukleinsäure in einem Vektor, einem Plasmid oder einem Cosmid enthalten ist.

10. Vektoren, Plasmide oder Cosmide enthaltend mindestens eine der Nukleinsäuren gemäß mindestens einem der Ansprüche 1 bis 3.

## Claims

1. A nucleic acid comprising coding and non-coding regions having the SEQ ID No. 4, wherein said coding regions code for the phospholipase A₁ (PLA₁) from *Tetrahymena thermophila* (SEQ ID No. 5), and said non-coding regions represent the upstream and downstream regions of this gene.

2. The nucleic acid according to claim 1, wherein said non-coding upstream and downstream regions have the SEQ ID Nos. 2 and 3, and the coding regions have the SEQ ID Nos. 1, 8 and/or 9.

3. A nucleic acid having one of the sequences SEQ ID Nos. 1, 2, 3, 8 or 9.

4. An N-terminal signal sequence of the PLA₁ (SEQ ID No. 5) according to claim 1, having the sequence SEQ ID No. 6.

5. A phospholipase A₁ protein (PLA₁) having the amino acid sequence according to SEQ ID No. 7.

6. Use of a nucleic acid according to claims 1 to 3 for the homologous or heterologous expression of recombinant proteins and peptides, and for homologous or heterologous recombination ("knock-out, "gene replacement").

7. A method for the homologous or heterologous expression of proteins and peptides and for the homologous or heterologous recombination ("knock-out, "gene replacement") in which ciliates are transfected with a nucleic acid according to any of claims 1 to 3.

8. The method according to claim 7, wherein said nucleic acids or parts thereof are combined with the enhancers, promoters, operators, origins, terminators, antibiotic resistances usual for the homologous or heterologous expression of proteins, or with other nucleic acids or DNA fragments or all kinds of sequences from viroids, viruses, bacteria, archezoans, protozoans, fungi, plants, animals or humans.

9. The method according to claim 7 and/or 8, wherein said nucleic acid is contained in a vector, a plasmid or a cosmid.

10. Vectors, plasmids or cosmids containing at least one of the nucleic acids according to at least one of claims 1 to 3.

## Revendications

1. Acide nucléique composé de domaines codants et non codants contenant SEQ. ID. NO. 4, dans lequel les domaines codants codent la phospholipase A₁ (PLA₁) de *Tetrahymena thermophila* (SEQ. ID. NO. 5) et les régions non codantes constituent les domaines en amont et en aval de ce gène.

2. Acide nucléique selon la revendication 1, dans lequel les domaines en amont et en aval non codants présentent SEQ. ID. NO. 2 et 3 et les régions codantes présentent SEQ. ID. NO. 1, 8 et/ou 9.

3. Acide nucléique avec l'une des séquences SEQ. ID. NO. 1, 2, 3, 8, ou 9.

4. Séquence signal N-terminale de la PLA₁ (SEQ. ID. NO. 5) selon la revendication 1, avec la séquence SEQ. ID. NO. 6.

5. Protéine phospholipase A₁ (PLA₁) contenant la séquence d'acides aminés selon SEQ. ID. NO. 7.

6. Utilisation d'un acide nucléique selon les revendications 1 à 3 pour l'expression homologue ou hétérologue de protéines et peptides recombinants, ainsi que pour la recombinaison homologue ou hétérologue ("knock-out", "remplacement de gène").

7. Procédé d'expression homologue ou hétérologue de protéines et de peptides, et de recombinaison homologue ou hétérologue ("knock-out", "remplacement de gène"), dans lequel des ciliées sont transfectés avec un acide nucléique selon l'une quelconque des revendications 1 à 3.

8. Procédé selon la revendication 7, dans lequel les acides nucléiques ou des fragments de ceux-ci sont combinés à des amplificateurs, des promoteurs, des opérateurs, des origines, des terminateurs, des antibiorésistances ou d'autres acides nucléiques ou fragments ou séquences d'ADN, habituels pour l'expression homologue ou hétérologue de protéines, de tous types de viroïdes, virus, bactéries, Archézoa, protozoaires, champignons, végétaux, animaux ou humains.

9. Procédé selon la revendication 7 et/ou 8, dans lequel l'acide nucléique est contenu dans un vecteur, un plasmide ou un cosmide.

10. Vecteurs, plasmides ou cosmides contenant au moins un des acides nucléiques selon au moins l'une quelconque des revendications 1 à 3.


### SEQUENCE LISTING

<110> Cilian AG
<120> Eine DNA-Sequenz des Enzyms Phospholipase A1 aus dem Ciliaten Tetrahymena und die Verwendung dieser.
<130> 020414wo ME/BM
<140> PCT/EP02/00578
   <141> 2002-01-22
<160> 9
<170> PatentIn Ver. 2.1
<210> 1
   <211> 963
   <212> DNA
   <213> Tetrahymena thermophila
<400> 1
   atgaacaaga ctctcatctt agctttagtt gttgttttgg ctttaactgc caccaccttg 60
   gttgctttcc acaaccactc tcacaacatc agagttgact aagaccccgc cactctcttc 120
   aagcaattca agcaaactta caataagaag tatgctgatg ctactttcga aacctacaga 180
   ttcggtgtct tcacccaaaa cttagaaatc gtcaagactg actctacttt cggtgtcacc 240
   taattcatgg acttaactcc tgctgaattc gctcaacaat tcctcacttt acacgaaaag 300
   gttaacagca ccgaagttta cagagcttaa ggtgaagcta ccgaagttga ctggactgcc 360
   aagggtaagg tcacccctgt taagaactaa ggttcttgtg gttcctgctg ggctttctcc 420
   accattggtg ccgttgaatc tgctctttgg attgctggtc aaggtgaata aaacactctt 480
   aaccttgctg aataagaata agttgactgt gctaagtccc ccaagtacga ctctgaaggt 540
   tgcaacggtg gttggatggt tgaaggtttc aagtacatca tcgacaacaa gatctcctaa 600
   actgctaact atccctacac tgctaaggat ggtaagtgca aggacacctc ttccttcaag 660
   aagttctcta tttctaagta cgctgaaatc ccctaaggtg actgcaactc cctcaactct 720
   gccttagaac aaggtcctat ctccgttgct gttgatgcca ccaacttcta attctacact 780
   tctggtgtct ttaaaaactg caaggccaac ctcaaccacg gtgtcctctt agttgccaac 840
   gttgactctt ctctcaagat caagaactcc tggggtcctt cttggggtga aaagggtttc 900
   atcagattag ctgccggtaa cacttgcggt gtctgcaatg ctgcctctta ccctattgtt 960
   tga                                                               963
<210> 2
   <211> 275
   <212> DNA
   <213> Tetrahymena thermophila
<400> 2
   aatatttatc aatgctactt ataattcttt tagtatgaga tatgatatgc tctttctctg 60
   ctagacttaa cttatgacat ttgaactttt aataaaagaa ttttttttat taaaaagcag 120
   agatttttaa tagaagaatc aatgactcat gaatttaata aagattttca agtgttttct 180
   aataccgact agctttataa attcacttat taatcaacga tataaaaatt atattaacaa 240
   atcaataaat aaaaaaataa ataaaaacaa aacaa 275
<210> 3
   <211> 171
   <212> DNA
   <213> Tetrahymena thermophila
<400> 3
   aaaaacataa tccaaattaa aaaaaattac tcaaaactga taatataaaa aattaatttt 60
   cataatttta atgtaaataa atacctttat atttgacgtt ttgtactcaa aataaattaa 120
   agttaacaaa ccatatttat ttaattctac ttttcaattt ttaaaaatat a 171
<210> 4
   <211> 1408
   <212> DNA
   <213> Tetrahymena thermophila
<400> 4
   aatatttatc aatgctactt ataattcttt tagtatgaga tatgatatgc tctttctctg 60
   ctagacttaa cttatgacat ttgaactttt aataaaagaa ttttttttat taaaaagcag 120
   agatttttaa tagaagaatc aatgactcat gaatttaata aagattttca agtgttttct 180
   aataccgact agctttataa attcacttat taatcaacga tataaaaatt atattaacaa 240
   atcaataaat aaaaaaataa ataaaaacaa aacaaatgaa caagactctc atcttagctt 300
   tagttgttgt tttggcttta actgccacca ccttggttgc tttccacaac cactctcaca 360
   acatcagagt tgactaagac cccgccactc tcttcaagca attcaagcaa acttacaata 420
   agaagtatgc tgatgctact ttcgaaacct acagattcgg tgtcttcacc caaaacttag 480
   aaatcgtcaa gactgactct actttcggtg tcacctaatt catggactta actcctgctg 540
   aattcgctca acaattcctc actttcacga aaaggttaac agcaccgaag tttacagagc 600
   ttaaggtgaa gctaccgaag ttgactggac tgccaagggt aaggtcaccc ctgttaagaa 660
   ctaaggttct tgtggttcct gctgggcttt ctccaccatt ggtgccgttg aatctgctct 720
   ttggattgct ggtcaaggtg aataaaacac tcttaacctt gctgaataag aataagttga 780
   ctgtgctaag tcccccaagt acgactctga aggttgcaac ggtggttgga tggttgaagg 840
   tttcaagtac atcatcgaca acaagatctc ctaaactgct aactatccct acactgctaa 900
   ggatggtaag tgcaaggaca cctcttcctt caagaagttc tctatttcta agtacgctga 960
   aatcccctaa ggtgactgca actccctcaa ctctgcctta gaacaaggtc ctatctccgt 1020
   tgctgttgat gccaccaact tctaattcta cacttctggt gtctttaaaa actgcaaggc 1080
   caacctcaac cacggtgtcc tcttagttgc caacgttgac tcttctctca agatcaagaa 1140
   ctcctggggt ccttcttggg gtgaaaaggg tttcatcaga ttagctgccg gtaacacttg 1200
   cggtgtctgc aatgctgcct cttaccctat tgtttgaaaa aacataatcc aaattaaaaa 1260
   aaattactca aaactgataa tataaaaaat taattttcat aattttaatg taaataaata 1320
   cctttatatt tgacgttttg tactcaaaat aaattaaagt taacaaacca tatttattta 1380
   attctacttt tcaattttta aaaatata 1408
<210> 5
   <211> 320
   <212> PRT
   <213> Tetrahymena thermophila
<900> 5
<210> 6
   <211> 110
   <212> PRT
   <213> Tetrahymena thermophila
<400> 6
   Met Asn Lys Thr Leu Ile Leu Ala Leu Val Gly Val Leu Ala Leu Thr
   1               5                   10                  15
   Ala Thr Thr Leu Val Ala Phe His Asn His Ser His Asn Ile Arg Val
               20                  25                  30
   Asp Gln Asp Pro Ala Thr Leu Phe Lys Gln Phe Lys Gln Thr Tyr Asn
           35                  40                  45
   Lys Lys Tyr Ala Asp Pro Thr Phe Glu Thr Tyr Arg Phe Gly Val Phe
       50                  55                  60
   Thr Gln Asn Leu Glu Ile Val Lys Thr Asp Ser Thr Phe Gly Val Thr
   65                  70                  75                  80
   Gln Phe Met Asp Leu Thr Pro Ala Glu Phe Ala Gln Gln Phe Leu Thr
                   85                  90                  95
   Leu His Glu Lys Val Asn Ser Thr Glu Val Tyr Arg Ala Gln
               100                 105                 110
<210> 7
   <211> 210
   <212> PRT
   <213> Tetrahymena thermophila
<400> 7
   Gly Glu Ala Thr Glu Val Asp Trp Thr Ala Lys Gly Lys Val Thr Pro
   1               5                   10                  15
   Val Lys Asn Gln Gly Ser Cys Gly Ser Cys Trp Ala Phe Ser Thr Ile
               20                  25                  30
   Gly Ala Val Glu Ser Ala Leu Leu Ile Ala Gly Gln Gly Glu Gln Asn
           35                  40                  45
   Thr Leu Asn Leu Ala Glu Gln Glu Leu Val Asp Cys Ala Lys Ser Pro
       50                  55                  60
   Lys Tyr Asp Ser Glu Gly Cys Asn Gly Gly Trp Met Val Glu Gly Phe
   65                  70                  75                  80
   Lys Tyr Ile Ile Asp Asn Lys Ile Ser Gln Thr Ala Asn Tyr Pro Tyr
                   85                  90                  95
   Thr Ala Lys Asp Gly Lys Cys Lys Asp Thr Ser Ser Phe Lys Lys Phe
               100                 105                 110
   Ser Ile Ser Lys Tyr Ala Glu Ile Pro Gln Gly Asp Cys Asn Ser Leu
           115                 120                 125
   Asn Ser Ala Leu Glu Gln Gly Pro Ile Ser Val Ala Val Asp Ala Thr
       130                 135                 140
   Asn Phe Gln Phe Tyr Thr Ser Gly Val Phe Lys Asn Cys Lys Ala Asn
   145                 150                 155                 160
   Leu Asn His Gly Val Leu Leu Val Ala Asn Val Asp Ser Ser Leu Lys
                   165                 170                 175
   Ile Lys Asn Ser Trp Gly Pro Ser Trp Gly Glu Lys Gly Phe Ile Arg
               180                 185                 190
   Leu Ala Ala Gly Asn Thr Cys Gly Val Cys Asn Ala Ala Ser Tyr Pro
           195                 200                 205
   Ile Val
       210
<210> 8
   <211> 330
   <212> DNA
   <213> Tetrahymena thermophila
<400> 8
   atgaacaaga ctctcatctt agctttagtt gttgttttgg ctttaactgc caccaccttg 60
   gttgctttcc acaaccactc tcacaacatc agagttgact aagaccccgc cactctcttc 120
   aagcaattca agcaaactta caataagaag tatgctgatg ctactttcga aacctacaga 180
   ttcggtgtct tcacccaaaa cttagaaatc gtcaagactg actctacttt cggtgtcacc 240
   taattcatgg acttaactcc tgctgaattc gctcaacaat tcctcacttt acacgaaaag 300
   gttaacagca ccgaagttta cagagcttaa                                  330
<210> 9
   <211> 633
   <212> DNA
   <213> Tetrahymena thermophila
<400> 9
   ggtgaagcta ccgaagttga ctggactgcc aagggtaagg tcacccctgt taagaactaa 60
   ggttcttgtg gttcctgctg ggctttctcc accattggtg ccgttgaatc tgctctttgg 120
   attgctggtc aaggtgaata aaacactctt aaccttgctg aataagaata agttgactgt 180
   gctaagtccc ccaagtacga ctctgaaggt tgcaacggtg gttggatggt tgaaggtttc 240
   aagtacatca tcgacaacaa gatctcctaa actgctaact atccctacac tgctaaggat 300
   ggtaagtgca aggacacctc ttccttcaag aagttctcta tttctaagta cgctgaaatc 360
   ccctaaggtg actgcaactc cctcaactct gccttagaac aaggtcctat ctccgttgct 420
   gttgatgcca ccaacttcta attctacact tctggtgtct ttaaaaactg caaggccaac 480
   ctcaaccacg gtgtcctctt agttgccaac gttgactctt ctctcaagat caagaactcc 540
   tggggtcctt cttggggtga aaagggtttc atcagattag ctgccggtaa cacttgcggt 600
   gtctgcaatg ctgcctctta ccctattgtt tga                              633